(19) Europäisches Patentamt
European Patent Office
Office européen des brevets

(11) **EP 2 606 928 B1**

(12) **EUROPÄISCHE PATENTSCHRIFT**

(45) Veröffentlichungstag und Bekanntmachung des Hinweises auf die Patenterteilung:
**03.03.2021   Patentblatt 2021/09**

(51) Int Cl.:
*A61M 5/31* *(2006.01)*          *A61M 5/34* *(2006.01)*

(21) Anmeldenummer: **12193370.9**

(22) Anmeldetag: **20.11.2012**

(54) **Spritze aus Borosilikatglas mit einer die Oberflächenrauhigkeit erhöhenden Konusbeschichtung**

Borosilicate glass syringe with a cone coating which increases the surface roughness

Seringue en borosilicate avec un revêtement de cône augmentant la rugosité de surface

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.12.2011   DE 102011089045**

(43) Veröffentlichungstag der Anmeldung:
**26.06.2013   Patentblatt 2013/26**

(73) Patentinhaber: **Schott AG**
**55122 Mainz (DE)**

(72) Erfinder:
• **Siebers, Dr. Friedrich**
**55283 Nierstein (DE)**
• **Henze, Dr. Inka**
**55268 Nieder-Olm (DE)**
• **Thürk, Dr. Jürgen**
**9008 St. Gallen/SG (CH)**

(74) Vertreter: **Blumbach · Zinngrebe Patentanwälte PartG mbB**
**Alexandrastraße 5**
**65187 Wiesbaden (DE)**

(56) Entgegenhaltungen:
**EP-A1- 0 370 683          EP-A1- 0 558 942**
**EP-A1- 0 585 830          EP-A1- 1 683 767**
**WO-A1-2010/150042          DE-A1- 19 834 801**
**DE-C1- 19 512 847          DE-C1- 19 721 737**
**US-A- 4 589 871**

**Beschreibung**

Die vorliegende Erfindung betrifft eine Spritze aus Borosilikatglas mit einer Beschichtung des Spritzenkonus zur Einstellung einer definierten Oberflächenrauhigkeit. Eine solche Beschichtung wird in Form einer keramischen Farbe auf den Konus aufgebracht und eingebrannt. Die dafür verwendete Farbmasse besteht aus einer Glasfritte, einem Rauhigkeitsadditiv und flüssigen organischen Bestandteilen, die sich beim Einbrennen verflüchtigen oder zersetzen. Die Glasfritte schmilzt und zerfließt beim Einbrennen, wohingegen das Rauhigkeitsadditiv beim Einbrennen der Schicht nicht aufschmilzt, im wesentlichen seine Form beibehält und in der aufgeschmolzenen Glasschicht eingebettete und an der Schichtoberfläche erhabene und strukturbildende Partikel darstellt. Die Glasmatrix der aufgeschmolzenen Schicht in welche die strukturbildenden Partikel eingebettet sind, wird auch als Glasfluss bezeichnet.

Bekanntermaßen werden anorganische Farben zur Beschichtung, auch Dekoration oder Bedruckung genannt, von Glasartikeln eingesetzt. Solche Farben enthalten eine oder mehrere Glasfritten und gegebenenfalls eine oder mehrere farbgebende Komponenten (Pigmente) in Mengen meist um 20 Gew.%. Die Glasfritte wird durch das Erschmelzen und anschließendes Abschrecken und Zerkleinern einer Glasmasse hergestellt. Die erhaltene Glasfritte wird auf Partikelgrößen bevorzugt < 30 μm gemahlen und mit den Pigmentpulvern vermischt.

Abgestimmt auf die zum Einsatz kommende Beschichtungstechnik werden die Pulverbestandteile mit spezifischen organischen Suspendiermitteln, z.B. Siebdrucköl, angepastet und auf das zu beschichtenden Glassubstrat aufgebracht. Das organische Suspendiermittel muss vor dem Sintern und Glattfließen des Glaspulvers rückstandsfrei ausbrennen, weil es ansonsten in der Beschichtung zu Bläschenbildung und verringerter Haftung auf dem Substrat kommt. Die Eigenschaften der organischen Komponenten sind mitbestimmend für die Auftragung, die Reproduzierbarkeit und die Standzeit der Pasten im Aufbringprozess. Maßgebliche Größen sind das Verdunstungs- und Trocknungsverhalten, die Viskosität und das Anpastverhältnis, d.h. das Verhältnis von festen zu flüssigen Bestandteilen bzw. von organischem Anteil zu anorganischem Pulver. Diese Größen bedingen unter anderem die im Aufbringprozess erzielbaren Schichtdicken, bei Einsatz der Siebdrucktechnik die Standzeit von Sieben oder von anderen Bedruckungselementen, sowie die Lagerzeiten von angepasteten Farben in Vorratsbehältern.

Im Einbrennprozess erweichen die Glaspartikel der Fritte, fließen glatt aus, umhüllen die Farbpigmente und stellen durch Diffusionsvorgänge die Haftung der eingebrannten Schicht auf dem Glassubstrat sicher. Die Glaspartikel der Fritte sind damit im Wesentlichen für die chemischen und physikalischen Eigenschaften der Dekoration oder Farbschicht nach dem Einbrand verantwortlich. Das Einbrennen der Farbe muss unterhalb der Verformungstemperatur des Glassubstrates stattfinden, damit eine unkontrollierte Verformung des Substrats das bedruckt oder dekoriert wurde vermieden wird. Deshalb ist es insbesondere für die Dekoration beispielsweise von Borosilikatgläsern mit Transformationstemperaturen von ca. 560°C notwendig, niedrigschmelzende Glasfritten zu verwenden, die unterhalb von 700°C eingebrannt werden können.

Die für die Beschichtung von Borosilikatgläsern bisher eingesetzten Glasfritten enthalten meist einen hohen Bleigehalt, der zur Senkung der Einbrenntemperatur zugesetzt wird. Neben den niedrigen Einbrenntemperaturen haben bleihaltige Glasfritten weitere technische Vorteile. Sie erlauben die Beschichtung von Borosilikatgläsern mit niedriger thermischer Ausdehnung von ca. 3 bis 6·10⁻⁶/K, ohne dass es zu Haftungsproblemen kommt. Weiterhin ermöglichen es bleihaltige Glasfritten Beschichtungen mit guter chemischer Beständigkeit gegen Säuren und Basen bereitzustellen. Dies ist für viele Anwendungen von Borosilikatgläsern ein Vorteil, da diese häufig aufgrund ihrer guten chemischen Beständigkeit eingesetzt werden.

Trotz dieser günstigen technischen Eigenschaften ist die Herstellung und Verarbeitung bleihaltiger Glasfritten u.a. bei der Schmelze und den Mahlungen problematisch. Die toxischen Eigenschaften von bleihaltigen Glasfritten erfordern hinsichtlich des Umgangs, der Verarbeitung und der Entsorgung der mit ihnen dekorierten Produkte eine gesonderte Behandlung. Durch neue und strengere Richtlinien, die die Verwendung von Blei in Glasfritten limitieren, besteht ein steigender Bedarf an bleifreien Glasfritten.

Kommerziell erhältliche bleifreie Farben für Einbrenntemperaturen von ca. 650°C haben thermische Ausdehnungen von mehr als 6·10⁻⁶/K und eignen sich daher eher für die Beschichtung von Kalknatronglas mit einer thermischen Ausdehnung von etwa 8 bis 9·10⁻⁶/K. Daher ist die thermische Dehnung solcher Farbbeschichtungen nicht in ausreichendem Maße an Borosilikatgläser angepasst, so dass in den beschichteten Bereichen leicht Schäden wie Risse, Abplatzungen und Schädigung des Substratglases entstehen können.

Eine Möglichkeit Spannungen zwischen einer Beschichtung und einem Borosilikatglassubstrat zu verringern ist das Aufbringen sehr dünner Schichtdicken, was jedoch den Nachteil einer geringeren Farbintensität hat. Auch die chemische Beständigkeit bleifreier Farben ist für viele Anwendungen unzureichend.

Die Anforderungen an eine Beschichtung eines Konus von Glasspritzen sind anders als die von sonst üblichen Farbbeschichtungen für z.B. dekorative Zwecke. Für Farbbeschichtungen mit guter Farbintensität sind in der Regel Schichtdicken von > 20 μm gewünscht. Die Schichtdicke von Konusbeschichtungen soll dagegen die Funktion sicherstellen und vorzugsweise zu Zwecken der Qualitätsüberwachung sichtbar sein, kann aber dünner sein. Während übliche, dekorative Farbbeschichtungen aus ästhetischen Gründen, sowie wegen der leichteren Reinigung glatt ausgebildet werden, muss die Konusbeschichtung über eine definierte Rauhigkeit verfügen. Diese definierte Rauhigkeit stellt sicher, dass auf den Konus einer Spritze aufgesetzte Adapter für den Transfer eines im Spritzenzylinder enthaltenen flüssigen Medikaments fest auf der Spritze bzw. deren Konus haften. Meist sind es Kunststoffadapter mit genormter Geometrie und daran befestigten Injektionsnadeln oder Schläuchen für den Transport der Flüssigkeit. Durch die mit der definierten Rauhigkeit einhergehende bessere Haftung des Adapters am Konus wird sichergestellt, dass ein Adapter durch den Innendruck oder beim Injizieren auftretende laterale Kräfte nicht gelöst wird. Ein auf den Konus aufgesetzter Spritzenadapter soll auch fest genug haften, damit er beim Herausziehen der Nadel nach einer Injektion fest mit dem Spritzenkörper verbunden bleibt. Auf der anderen Seite darf die Rauhigkeit aber nicht zu groß gewählt werden, weil dann die Gefahr mangelnder Dichtigkeit besteht. Mangelnde Dichtigkeit kann dazu führen, dass durch den beim Injizieren erforderlichen Druck ein Teil der medizinischen Flüssigkeit zwischen Adapter und Spritzenkonus herausgedrückt wird und verloren geht, wobei die Umgebung durch die herausgedrückten Tropfen kontaminiert wird. Weiter werden vorgefüllte Fertigspritzen mitsamt medizinischer Flüssigkeit nach dem Befüllen mit Verschlusskappen am Konus abgedichtet und gelagert. Bei dieser Lagerung ist eine gute Dichtheit wichtig, damit die medizinische Flüssigkeit sicher aufbewahrt wird und hermetisch gegen die Umgebung und Atmosphäre abgeschirmt wird.

Für Pharmaverpackungsmittel bestehen verschärfte Forderungen hinsichtlich Sicherheit, toxischer Belastung und Umweltfreundlichkeit. So fordert die EU Direktive EU 94/62/EC ebenso wie die in den Vereinigten Staaten von Amerika gültige Verpackungsordnung Coneg den Gehalt der Elemente Pb, Cd, Hg und $Cr^{VI}$ in der Summe auf weniger als 100 ppm (entspricht 0,01 Gew. %) pro Artikel zu begrenzen. Mit Konusbeschichtungen, die auf bleihaltigen Glasfritten basieren, wird dieser Grenzwert, insbesondere bei kleinen und leichten Glasspritzen, leicht überschritten.

Wichtig für die gewünschte Funktion von Konusbeschichtungen ist daher insbesondere eine gute Haftung der Beschichtung, ohne dass es zu Rissen, Abplatzungen der Beschichtung und Festigkeitserniedrigungen des Glassubstrats kommt. Dies setzt eine Anpassung der thermischen Ausdehnung der Konusbeschichtung auf den Glastyp der Spritze voraus. Aus Unterschieden in der thermischen Ausdehnung bedingte Spannungen müssen so minimiert werden, dass die genannten Beschädigungen vermieden werden.

Wünschenswert ist auch eine gute chemische Beständigkeit der Konusbeschichtung, damit Reinigungsvorgänge, wie z.B. das Autoklavieren oder Waschen der Glasspritze mittels meist sauerer oder basischen Reinigungsmitteln, die Konusbeschichtung nicht angreifen. Auch soll sich die eingestellte Rauhigkeit der Beschichtungsoderfläche durch chemischen Angriff nicht verändern.

Damit der Glaskörper einer Spritze aus Borosilikatglas beim Einbrennen nicht verformt, muss die Glasfritte in der Farbe weiterhin eine niedrige Einbrenntemperatur von < 700°C besitzen.

Konusbeschichtungen für Spritzen werden in US 4589871 A beschrieben. Die Spritzenkörper können aus Metall, Plastik, Glas und Keramik bestehen. Als Rauhigkeitsadditive werden Pulver aus Keramik, Glas, Metall oder Kombinationen davon beschrieben, die einem Öl zugegeben werden. Diese Suspension wird auf den Spritzenkonus aufgedruckt und die flüssigen Bestandteile teilweise in einem Ofen verdampft, während die Partikel auf dem Konusoberfläche haften bleiben sollen. Es werden jedoch keine Hinweise für die Konusbeschichtung von Spritzen des Borosilikatglastyps mit ihrer niedrigen thermischen Ausdehnung gegeben. Es werden auch keine Angaben gemacht, wie das Rauhigkeitsadditiv beschaffen sein muss. Es ist ferner zu erwarten dass die Haftkraft der aufgebrachten Partikel am Konus zu wünschen übrig lässt. Ferner werden auch keine Rauhigkeitswerte für eine optimale Funktion der Beschichtung genannt WO2010/150042 beschreibt auch ein Aufrauen zumindest eines Teilbereiches des Spritzenkonus.

US 5851201 A beschreibt eine Spritze, die zur definierten Einstellung der Haftungskräfte außen im Konusbereich mit einer texturierter Oberfläche versehen ist. Die Rauhigkeit wird durch Einprägen der Textur in die Oberfläche des Spritzenkonus erzeugt.

Als Alternative zum Aufbringen einer Beschichtung mit definierter Rauhigkeit kann die Glasspritze im Konusbereich über Schleifwerkzeuge oder Sandstrahlen angeraut werden. Es ist jedoch schwierig bzw. aufwendig auf diesem Wege eine definierte Rauhigkeit bereitzustellen. Außerdem erzeugen diese Verfahren gerade bei Spritzen sehr unerwünschte Verunreinigungen durch Abrieb und Partikel und machen aufwändige Reinigungsschritte erforderlich.

Es ist Aufgabe der Erfindung eine Glasspritze aus Borosilikatglas mit einer Konusbeschichtung bereitzustellen, die mit ausreichender Dichtheit verschlossen werden kann und auf deren Konus aufgesetzte Adapter zuverlässig halten. Dabei soll die Haftung von aufgesetzten Adaptern erhöht werden und andererseits eine sichere Dichtigkeit gewährleistet sein. Die Spritzen sollen außerdem wirtschaftlich herstellbar sein und die regulatorischen Anforderungen für die Pharmaindustrie erfüllen.

Da Borosilikatgläser über eine besonders gute chemische Beständigkeit verfügen und damit gegenüber einer medizinischen Flüssigkeit auch bei längeren Lagerzeiten weitgehend inert sind, soll die Konusbeschichtung auf Spritzen aus diesem Glastyp gerichtet sein. Das Borosilikatglas vom Typ FIOLAX® der Firma SCHOTT AG, vormals SCHOTT-Rohrglas GmbH, ist ein Standard für Pharmaverpackungsmittel und verfügt abhängig vom Typ über eine thermische Ausdehnung im Temperaturbereich von 20 bis 300 °C von 4,9 bis 5,5·$10^{-6}$/K. Die Konusbeschichtung soll u.a. für diese Borosilikatgläser geeignet sein.

Diese Aufgaben werden durch eine Spritze mit einer Konusbeschichtung nach Anspruch 1 gelöst.

Die Glasspritze soll aus einem Borosilikatglas bestehen. Borosilikatglas zeichnet sich durch eine hohe chemische Resistenz aus, ist gut sterilisierbar und verfügt über ausgezeichnete Barriereeigenschaften gegenüber Sauerstoff, um das flüssige Medikament zu schützen. Aufgrund seines niedrigen Alkali-Gehalts verhält es sich weitgehend inert gegen das Medikament, auch bei längeren Lagerungs- und Kontaktzeiten. Die thermische Ausdehnung des Borosilikatglases ist vor allem abhängig von den Alkaligehalten. Für inertes Verhalten und gute Verarbeitungseigenschaften in der Heißformgebung mit Flammenbrennern soll das Borosilikatglas der Spritze eine thermische Ausdehnung zwischen 20 und 300 °C von weniger als $6 \cdot 10^{-6}$/K, bevorzugt höchstens $5,5 \cdot 10^{-6}$/K und besonders bevorzugt weniger als $5 \cdot 10^{-6}$/K besitzt. Bei hohen Ansprüchen an Pharmaverpackungsmitteln ist es daher von Vorteil ein Borosilikatglas wie das bewährte FIOLAX® der Firma SCHOTT AG einzusetzen. Dieses verfügt über eine niedrige thermische Ausdehnung im Temperaturbereich von 20 bis 300 °C von 4,9 bis $5,5 \cdot 10^{-6}$/K. Die Transformationstemperatur ($T_g$) dieser Gläser beträgt, wie für Borosilikatgläser typisch, 565 °C. Um eine Verformung einer Glasspritze aus diesem Glastyp auszuschließen, erfolgt der Einbrand der Konusbeschichtung unterhalb 700 °C und bevorzugt unterhalb von 660 °C. Für das Glattfließen der Konusbeschichtung und die sichere Haftung auf dem Spritzenkonus enthält die Beschichtung 70-99 Gew.% einer niedrig schmelzenden Glasmatrix, die bei einer Temperatur von unterhalb 700 °C eine Viskosität von $\leq 10^6$ dPas erreicht. Dieser Viskositätswert gewährleistet bei mittleren Korngrößen der Glasfritte von ca. 3 $\mu$m das Glattfließen der Glaspulver beim Einbrennen und die Erzeugung einer auf dem Substrat gut haftenden Schicht. Bevorzugt wird diese Viskosität beim Einbrennen schon unterhalb 660 °C erreicht, da bei Verwendung von besonders fein gemahlenem Glaspulver die Einbrenntemperaturen unter 660 °C erniedrigt werden können.

Um die Funktion der sicheren Haftung und Dichtigkeit von aufgesetzten Adaptern und Verschlusskappen sicherzustellen besteht die Konusbeschichtung neben der Glasfritte aus 1 bis 30 Gew.% eines pulverförmigen Rauhigkeitsadditivs. Bevorzugt ist ein Gehalt von 4 bis 25 Gew.%. Während die Glasfritte für das Glattfließen und die Haftung der Konusbeschichtung auf dem Glassubstrat verantwortlich ist, wird über das zugesetzte Additiv eine definierte Rauhigkeit eingestellt. Die Partikel des Additivs sollen beim Einbrennen selbst nicht glatt fließen, sondern ihre ursprüngliche Form behalten.

Die Konusbeschichtung ist innerhalb technisch üblicher Grenzen frei von den bedenklichen Elementen Pb, Cd, Hg und Cr$^{VI}$. Das bedeutet, dass Komponenten mit diesen Elementen nicht absichtlich als Bestandteil der Beschichtung bzw. der Ausgangsfarbe zugesetzt werden und diese Elemente lediglich als mit vertretbarem Aufwand unvermeidbare Verunreinigungen Eingang finden.

Bezogen auf eine gesamte Spritze liegt die Konzentration dieser Elemente durch Verunreinigungen in der Summe unterhalb von 1000 ppm, bevorzugt unterhalb von 100 ppm.

Für optimale Funktion wird der Rauhigkeitswert der Konusbeschichtung auf Werte von $R_q$(rms) = 0,3 bis 2 $\mu$m und bevorzugt 0,5 bis 1,8 $\mu$m eingestellt. Dabei entspricht der $R_q$(rms)-Wert (rms: root mean square) dem Mittelwert der Höhenabweichungen z(x) gemessen von der mittleren Höhenfläche im Messbereich und wird gemäß der nachfolgend angegebenen Formel bestimmt.

$$R_q = \sqrt{\frac{1}{L} \int_0^L z^2(x)\,dx}$$

Dabei steht x für die laterale Ortskoordinate entlang der Messstrecke von 0 bis L.

Die Rauhigkeit korrespondiert mit der Haftung des Adapters. Besonders vorteilhafte Werte für die Haftung und die Dichtigkeit werden erreicht mit Werten von $R_q$(rms) = 0,5 bis 1,8 $\mu$m. Mit diesen Werten wird ein guter Kompromiss zwischen Haftung und Dichtigkeit erreicht.

Für die Funktion der Konusbeschichtung hinsichtlich Haftung und Dichtigkeit ist es vorteilhaft, wenn die mittlere Schichtdicke 0,5 bis 20 $\mu$m beträgt. Bei Schichtdicken von etwa 0,5 $\mu$m wird mitunter beobachtet, dass die Konusbeschichtung den Spritzenkonus nicht überall vollständig bedeckt. Innerhalb beschichteter Flächen gibt es dann inselartige Teilbereiche, die frei von Beschichtung sind. Es hat sich gezeigt, dass eine nur stellenweise nicht vollständige Beschichtung für die Funktion nicht kritisch ist. Unterhalb einer mittleren Schichtdicke von 0,5 $\mu$m wird die Konusbeschichtung jedoch visuell unauffällig. Das bedeutet, dass bei der visuellen Qualitätskontrolle Unterschiede in der Schichtdicke kaum festgestellt werden können. Damit nehmen auch die möglichen Abweichungen hinsichtlich der Haftungskräfte zu. Die mittlere Schichtdicke der Konusbeschichtung soll daher mindestens 0,5 $\mu$m betragen. Bei mittlerer Schichtdicke der Konusbeschichtung oberhalb 20 $\mu$m sind die mechanischen Spannungen zwischen der Beschichtung und der Glasspritze aus Borosilikatglas erhöht, was zu Rissen oder Abplatzungen führen kann. Die Festigkeit der Glasspritze im Konusbereich kann durch diese Schädigungen in unzulässiger Weise erniedrigt werden. Besonders vorteilhaft sind mittlere Schichtdicken von 2 bis 15 $\mu$m, weil Schichten mit dieser Dicke visuell gut sichtbar sind und die mechanischen Spannungen verringert sind.

Die Rauhigkeit der Konusbeschichtung hängt insbesondere ab von der Art, Menge und Korngrößenverteilung

der eingesetzten Rauhigkeitsaddditive. Die mittlere Korngröße $d_{50}$ des Rauhigkeitsadditivs wird vorteilhaft auf Werte von 0,2 bis 5 $\mu$m eingestellt. Die Wahl der Korngröße hängt davon ab, welche Schichtdicke der Konusbeschichtung im Aufbringprozess eingestellt wird. Bei größeren Schichtdicken ist es zur Erzeugung des gewünschten Rauhigkeitswertes vorteilhaft, wenn die Korngröße im oberen Bereich des angegebenen Intervalls gewählt wird. Dies ist darin begründet, dass bei höheren Schichtdicken die Körner, bzw. Partikel des Additivs stärker vom Glasfluss umschlossen werden. Maßgeblich für die Wahl der Korngröße des Additivs ist das Erreichen der gewünschten Rauhigkeitswerte. Nach dem Glattfließen der auf den Konus aufgebrachten Beschichtung während des Einbrennens ragen die Partikel des Rauhigkeitsadditivs aus der vom Glasfluss gebildeten Glasmatrix teilweise heraus und bilden dadurch raue Oberflächenstruktur. Die Partikel des Rauhigkeitsadditivs werden daher auch als strukturbildende Partikel bezeichnet.

Es sind verschiedenen Arten von Rauhigkeitsadditiven möglich. Es können keramische Pulver, wie z.B. Korund, Zirkonsilikat, Schwerspat, $TiO_2$, $ZrO_2$, verwendet werden. Ebenso ist es möglich ein Hartglas, wie z.B. Kieselglas oder ein anderes Glas mit deutlich höherem Schmelzpunkt als dem der Glasfritte und mit hoher Viskosität, als pulverförmiges Rauhigkeitsadditiv zuzusetzen. Vorteilhaft ist es wenn ein Additiv zugesetzt wird, dessen thermische Ausdehnung nicht größer ist als die thermische Ausdehnung des verwendeten Glasflusses. Auf diese Weise kann über den Zusatz des Additivs die thermische Ausdehnung der resultierenden gesamten Beschichtung verringert werden. Dadurch wird eine bessere Anpassung der thermischen Ausdehnung an das Substrat erreicht, das aus niedrig dehnendem Borosilikatglas besteht. Vorteilhaft ist es, wenn die thermische Ausdehnung des Rauhigkeitsadditivs im Temperaturbereich von 20 bis 300 °C weniger als $8 \cdot 10^{-6}$/K beträgt und besonders vorteilhaft weniger als $5 \cdot 10^{-6}$/K beträgt. Solche vorteilhaften Additive bestehen z.B. aus Cordierit, Kieselglas, Eukryptit, Zirkonsilikat und Mullit.

Geeignete Glasfritten für die Beschichtung von Borosilikatgläsern mit niedrigem thermischen Ausdehnungskoeffizient verfügen über eine thermische Ausdehnung zwischen 20 und 300 °C von weniger als $7,5 \cdot 10^{-6}$/K und bevorzugt weniger als $7 \cdot 10^{-6}$/K. Mit diesen Glasflüssen sind gut haftende Beschichtungen bis zu etwa 20 $\mu$m Schichtdicke möglich, ohne dass es zu Rissen und Abplatzungen kommt, was für den Einsatzzweck im pharmazeutischen Bereich sehr unerwünscht wäre. Die thermische Ausdehnung soll dabei zur Anpassung an das Glassubstrat größer als $4 \cdot 10^{-6}$/K sein.

Für ein gutes Glattfließen und Haften der Beschichtung auf dem Borosilikatglas soll die Korngröße der Glasfritte möglichst fein sein. Um den technischen Aufwand bei der Feinmahlung nicht zu groß zu gestalten, wird bis auf eine mittlere Korngröße von ca. 1 bis 5 $\mu$m gemahlen. Bevorzugterweise liegt die mittlere Korngröße der Glaspulver bei ca. 2 bis 4 $\mu$m.

Die Forderungen die an die Glasfritte gestellt werden, wie umweltfreundliche Zusammensetzung, Anpassung an die aus Borosilikatglas bestehende Glasspritze hinsichtlich Einbrenntemperatur und Anpassung des thermischen Ausdehnungskoeffizienten werden von zwei Glassystemen vorteilhaft bedient.

Der Einsatz des kristallchemisch verwandten und toxikologisch unbedenkliche Bi als Ersatz für Pb führt zu dem $Bi_2O_3$-$B_2O_3$-$SiO_2$-Glassystem. Dieses Glassystem enthält als Hauptbestandteile 40 - 75 Gew.% $Bi_2O_3$, 3 - 20 Gew.% $B_2O_3$ und 10 - 30 Gew.% $SiO_2$, die das Glasgerüst bilden. Dafür sind Mindestgehalte von 40 Gew.% $Bi_2O_3$, 10 Gew.% $SiO_2$ und 3 Gew.% $B_2O_3$ erforderlich. Höhere Gehalte an $Bi_2O_3$ als 75 Gew.% führen zu einer unzulässigen Erhöhung der thermischen Ausdehnung. $B_2O_3$-Gehalte oberhalb 20 Gew.% sind nachteilig hinsichtlich der chemischen Beständigkeit. $SiO_2$ ist in Gehalten von maximal 30 Gew.% enthalten. Höhere Gehalte würden die Einbrenntemperatur über die für die Verformung von Glasspritzen aus Borosilikatglas geltenden Grenzen erhöhen.

Besonders vorteilhaft ist es wenn die Glasfritte im Wesentlichen folgende Komponenten (in Gew.% auf Oxidbasis) aufweist oder daraus besteht:

| | |
|---|---|
| $Bi_2O_3$ | 55 - 70 Gew.% |
| $B_2O_3$ | 5 - 15 Gew.% |
| $SiO_2$ | 15 - 30 Gew.% |
| $\sum Li_2O + Na_2O + K_2O$ | 1 - 5 Gew.% |
| $\sum MgO + CaO + SrO + BaO$ | 0 - 4 Gew.% |
| $ZnO$ | 0 - 4 Gew.% |
| $Al_2O_3$ | 0 - 5 Gew.% |
| $\sum TiO_2 + ZrO_2$ | 0 - 5 Gew.% |

Mit dieser Zusammensetzung sind die an die Konusbeschichtung gestellten Forderungen hinsichtlich niedriger thermischer Ausdehnung, angepasst an Borosilikatglas, sowie niedrige Einbrenntemperatur weiter verbessert. Die Gehalte an Alkalien und Erdalkalien erniedrigen die Viskosität und damit die Einbrenntemperatur, dürfen aber nicht höher gewählt werden, weil sich sonst die thermische Ausdehnung des Glassflussanteils bzw. der Glasmatrix in der Beschichtung unzulässig erhöht. Die Zusätze an $Al_2O_3$, $TiO_2$ und $ZrO_2$ verbessern die Glasstabilität und wirken einer unerwünschten Kristallisation entgegen. Höhere Gehalte als die angegebenen führen jedoch zu einer Erhöhung der Viskosität und der Einbrenntemperatur. Der Gehalt an ZnO ist begrenzt, weil diese Komponente sonst zu einer unerwünschten Kristallisation führen kann.

Ein alternatives zweites Glassystem dass die Anforderungen, die an die Glasfritte gestellt werden, erfüllen kann, ist das ZnO-B$_2$O$_3$-SiO$_2$-Glassystem. Diese drei Komponenten bilden als Hauptbestandteile das Glasgerüst. Diese Hauptbestandteile sind in Anteilen von 15 - 48 Gew.% ZnO, 8 - 40 Gew.% B$_2$O$_3$ und 8 - 52 Gew.% SiO$_2$ enthalten. Mit diesem Glassystem ist es möglich, vergleichsweise niedrige thermische Ausdehnungskoeffizienten von ca. $6 \cdot 10^{-6}$/K bei 20 und 300 °C zu erhalten.

Bei höheren SiO$_2$-Gehalten als 52 Gew.% verbessert sich die chemische Beständigkeit weiter, allerdings erhöhen sich die Viskosität und die Einbrenntemperatur in unzulässiger Weise. Nachteilig bei niedrigen SiO$_2$-Gehalten unter 8 Gew.% ist die verringerte chemische Beständigkeit gegenüber Säuren und Basen, sodass es hier immer gilt, über die Zusammensetzung einen Kompromiss zu finden, der den Anforderung gerecht wird.

Der Mindestgehalt an ZnO soll 15 Gew.% und der Mindestgehalt an B$_2$O$_3$ 8 Gew.% betragen. Dies ist erforderlich, weil sich sonst die Einbrenntemperaturen zu stark erhöhen. Höhere Gehalte als 48 Gew.% ZnO und 40 Gew.% B$_2$O$_3$ sind für die chemische Beständigkeit nachteilig und führen zu Glasfritten, die beim Einbrand sehr anfällig für eine unerwünschte Kristallisation sind.

Eine Kristallisation der Glasfritte ist unerwünscht weil diese Kristallisation von den Oberflächen ausgeht und die entstehende Kristallschicht das Sintern und Glattfließen des Glaspulvers verhindert. Kristallisationsanfällige Glasfritten führen daher meist zu porösen Schichten, die nicht gut haften und leicht mechanisch entfernbar sind. Da sich die Kristallisation aufgrund ihrer starken Temperatur- und Korngrößenabhängigkeit nicht gut steuern lässt, ist sie für die Einstellung einer definierten Rauhigkeit weniger geeignet.

Besonders vorteilhaft ist eine Zusammensetzung der Glasfritte die im wesentlichen die folgenden Komponenten (in Gew.% auf Oxidbasis) aufweist oder aus ihnen besteht:

| | |
|---|---|
| ZnO | 17 - 35 Gew.% |
| B$_2$O$_3$ | 10 - 30 Gew.% |
| SiO$_2$ | 20 - 50 Gew.% |
| $\sum$ Li$_2$O + Na$_2$O + K$_2$O | 1 - 15 Gew.% |
| $\sum$ MgO + CaO + SrO + BaO | 0 - 3 Gew.% |
| Al$_2$O$_3$ | 0 - 3 Gew.% |
| $\sum$ TiO$_2$ + ZrO$_2$ | 0 - 7 Gew.% |

Mit dieser Zusammensetzung sind die angestrebten Eigenschaften hinsichtlich niedriger thermischer Ausdehnung, angepasst an Borosilikatglas, sowie niedrige Einbrenntemperatur weiter verbessert. Die Gehalte an Alkalien erniedrigen die Viskosität und damit die Einbrenntemperatur, dürfen aber nicht höher gewählt werden, weil sich sonst die thermische Ausdehnung der Glasfritte unzulässig erhöht. Die Zusätze an Erdalkalien sowie Al$_2$O$_3$, TiO$_2$ und ZrO$_2$ verbessern die Glasstabilität und wirken einer unerwünschten Kristallisation entgegen. Höhere Gehalte als die angegebenen führen zu einer unerwünschten Erhöhung der Viskosität und der Einbrenntemperatur.

Eine weitere Aufgabe der Erfindung besteht darin eine Glasspritze aus Borosilikatglas mit einer umweltfreundlichen Konusbeschichtung bereitzustellen, welche eine ausreichende Haftung und Dichtigkeit von aufgesetzten Adaptern und Verschlusskappen gewährleistet. Für die Funktion der Glasspritze hat es sich als vorteilhaft erwiesen, wenn die Konusbeschichtung rotationssymmetrisch umlaufend ist und eine Breite von 2 bis 10 mm besitzt. Diese Breite ist für die üblichen Verschlusskappen und Adapter von Vorteil. Kleinere Breiten können zu unerwünschten Abweichungen bei der Dichtigkeit und der Haftung führen. Durch die Größe des Spritzenkonus bei üblichen Glasspritzen ist die Breite der Beschichtung auf maximal 10 mm begrenzt. Größere Breiten führen zu einem schwierigeren Beschichtungsprozess. Die umlaufende Beschichtung wird im Aufbringprozess aufgetragen, beispielsweise durch Druckwalzen oder Druckbänder, wobei die Glasspritze beim Bedruckungsvorgang gedreht wird, um eine einheitliche Schichtdicke zu gewährleisten. In vorteilhafter Weise ist die Konusbeschichtung ohne Unterbrechung umlaufend ausgeführt, damit es nicht zu Dichtigkeitsproblemen kommt. Für eine visuelle Qualitätskontrolle soll die Beschichtung bevorzugt mit dem bloßen Auge sichtbar sein. Sie kann dabei transparent bis transluzent sein, was in der Regel durch den Sinterprozess und die Brechungsunterschiede zwischen Glasfritte und Rauhigkeitsadditiv sowie die Rauhigkeit der Konusbeschichtung gewährleistet ist.

Um die Sichtbarkeit zu erhöhen, kann der Glasfritte neben dem Rauhigkeitsadditiv bis zu 5 Gew.% eines Weißpigmentes, z.B. TiO$_2$, zugesetzt werden. Eine erfindungsgemäß beschichtete Spritze stellt damit nicht Funktionen der Spritze sicher, sondern durch die Geometrie und das Aussehen der Beschichtung werden auch Maßnahmen zur Qualitätsüberwachung bei der Herstellung ermöglicht.

Die beschichtete Glasspritze enthält bezogen auf ihr Gesamtgewicht in der Summe weniger als 100 ppm der toxikologisch bedenklichen Elemente Pb, Cd, Hg, Cr$^{VI}$ und erfüllt somit die gesetzlichen Forderungen, die an Pharmaverpackungsmittel gestellt werden. Die Beschichtung der Glasspritze besteht aus einem niedrigschmelzenden Glas mit einer Viskosität von ≤ $10^6$ dPas unterhalb 700 °C sowie 1 - 30 Gew.% eines Rauhigkeitsadditivs. Bei diesem Viskositätswert, der für das Sintern und Glattfließen erreicht werden soll, werden die erforderlichen niedrigen Einbrandtemperaturen sichergestellt. Vorteilhaft ist es, wenn dieser Viskositätswert schon unterhalb 660 °C erreicht wird, weil dann die Einbrandtemperatur weiter abgesenkt werden kann. Der Anteil von 1 bis 30 Gew.% des Rauhigkeitsadditivs stellt die definierte Rauhigkeit sicher.

Weitere Eigenschaften der auf der Glasspritze befindlichen Konusbeschichtung werden entsprechend den vorangegangenen Ausführungen gewählt. So ist eine mittlere Schichtdicke der Konusbeschichtung von 0,5 bis 20 μm vorteilhaft und eine mittlere Korngröße $d_{50}$ der Rauhigkeitsadditive als strukturbildende Partikel von 0,2 bis 5 μm. Weiterhin ist eine niedrige thermische Ausdehnung der Rauhigkeitsadditive vorteilhaft, ebenso wie eine thermische Ausdehnung der Glasflussanteils bzw. der Glasmatrix der Beschichtung von < 7, $5·10^{-6}$/K und bevorzugt < $7·10^{-6}$/K im Temperaturbereich zwischen 20 und 300 °C.

Die Glasspritze besteht aus einem Borosilikatglas das gegenüber den flüssigen Medikamenten besonders inert ist. Da dies niedrige Alkaligehalte des Borosilikatglases erfordert, geht es einher mit niedriger thermischer Ausdehnung von < $6·10^{-6}$/K, bevorzugt höchstens $5,5·10^{-6}$/K und besonders bevorzugt < $5·10^{-6}$/K. Dies erhöht zwar die Anforderungen, die an die Anpassung der thermischen Ausdehnung der Konusbeschichtung gestellt werden, ist aber für das inerte Verhalten ein großer Vorteil, da auch bei länger Lagerung und langen Kontaktzeiten nur sehr wenige Alkaliionen in Lösung gehen. Unterhalb einer thermischen Ausdehnung des Borosilikatglases von $4,5·10^{-6}$/K wird die Anpassung der thermischen Ausdehnung der Konusbeschichtung erschwert, sodass es zu Spannungen und Rissen oder Haftungsproblemen der Konusbeschichtung kommt.

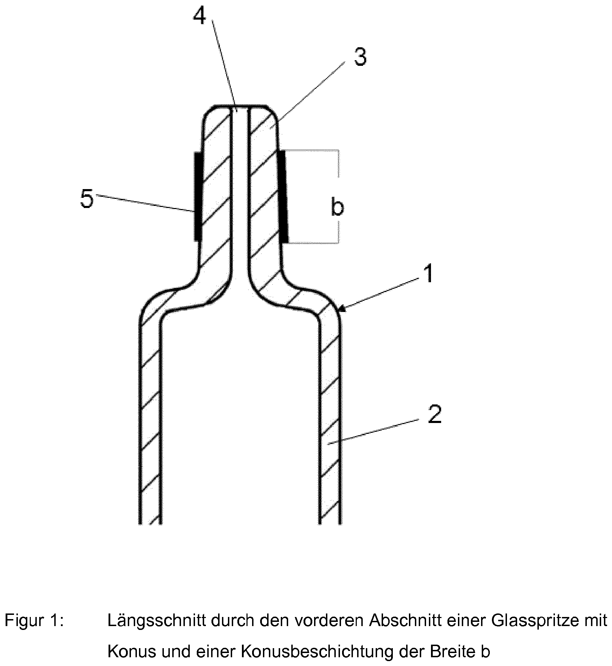

Fig. 1 zeigt den vorderen Abschnitt einer Glasspritze 1 mit Zylinder 2, Konus 3, Auslasskanal 4 und umlaufender Konusbeschichtung im Längsschnitt. Eine typische Abmessung der umlaufenden Konusbeschichtung wäre eine Breite b von 5 mm. Ist die Beschichtung 5 wie dargestellt, im zentralen, mittleren Bereich des Glaskonus angeordnet, ist dies für die Funktion und Handhabung bei dem Aufsetzen von Adaptern und Dichtkappen besonders günstig.

Bei dieser Art der Ausführung der Konusbeschichtung sind Abweichungen in der Rauhigkeit, z.B. durch schwankende Einbrenntemperaturen oder Schichtdicken visuell unter geeigneten Beleuchtungsverhältnissen sehr gut sichtbar und können für die abschließende Qualitätskontrolle der beschichteten Spritzen genutzt werden.

Die vorliegende Erfindung wird mit Hilfe der folgenden Beispiele weiter verdeutlicht.

Die Glaszusammensetzungen für die Glasfritten der Tabelle 1 wurden aus üblichen technischen Gemengerohstoffen erschmolzen. Nach dem Schmelzen und Homogenisieren der Glasschmelze bei Temperaturen um 1550 °C wird diese zur Erzeugung eines leicht mahlbaren Granulates in kaltes Wasser gegossen und abgeschreckt. Die erhaltenen mahlfähigen Glasgranulate werden zu Pulvern einer mittleren Korngröße von 2 bis 4 μm vermahlen. In der Tabelle 1 sind erfindungsgemäße Beispiele Nr. 1-3 und ein bleihaltiges Vergleichsglas Nr. 4 dargestellt. Die Tabelle enthält auch Eigenschaften, die an diesen Gläsern gemessen wurden, wie Dichte, thermische Ausdehnung zwischen 20 und 300 °C, Transformationstemperatur $T_g$, sowie die Temperatur, bei der die Viskosität des Glases $10^6$ dPas beträgt. Die chemische Beständigkeit ist, angelehnt an die Norm DIN ISO 4794, über den Masseverlust in mg/dm$^2$ nach einem einstündigen Angriff durch 2 mol/l HCl Säure bei 23 °C gemessen.

Tabelle 1:

| Zusammensetzungen und Eigenschaften der Gläser für die Fritte Nr. 1 - 3 und des Vergleichsglases Nr. 4 | | | | |
|---|---|---|---|---|
| Glas | 1 | 2 | 3 | 4 |
| Zusammensetzung | | | | |
| (Gew.%) | | | | |
| $Al_2O_3$ | 0,4 | 3,3 | 1,2 | 0,3 |
| $B_2O_3$ | 14,2 | 7,2 | 34,7 | 1,6 |
| $Bi_2O_3$ | | 64,2 | | |
| CaO | | 0,2 | | 0,1 |
| $K_2O$ | | 0,5 | | 0,1 |
| $Li_2O$ | 2,0 | 1,3 | 1,9 | 3,1 |
| MgO | | 0,1 | | 0,2 |
| $Na_2O$ | 8,6 | | | 2,5 |
| PbO | | | | 48,6 |

(fortgesetzt)

| Glas | 1 | 2 | 3 | 4 |
|---|---|---|---|---|
| SiO$_2$ | 49,2 | 21,0 | 8,9 | 41,8 |
| TiO$_2$ | 1,9 | 0,1 | | 1,7 |
| ZnO | 20,3 | 0,1 | 45,5 | |
| ZrO$_2$ | 3,4 | 2,0 | 7,8 | |
| Eigenschaften: | | | | |
| Dichte [g/cm$^3$] | 2,807 | 4,522 | 3,364 | 3,841 |
| Therm. Ausdehnung von 20 bis 300 °C [10$^{-6}$/K] | 7,1 | 7,3 | 5,2 | 8,7 |
| T$_g$[°C] | 489 | 445 | 514 | 405 |
| Temperatur bei Viskosität η = 10$^6$ dPas [°C] | 675 | 615 | 645 | 600 |
| Chem. Beständigkeit: 2 mol/l HCl (1h, 23 °C) [mg/dm$^2$] | 145 | 230 | 2990 | 1 |

Glas Nr. 3 zeigt bereits einen vergleichsweise starken chemischen Angriff, der für Anwendungen mit hohen Ansprüchen hinsichtlich der Beständigkeit unzureichend sein kann.

Die Glasfritten aus Tabelle 1 werden zu Pasten bzw. Druckfarben verarbeitet. Wie in Tabelle 2 aufgeführt, werden die pulverförmigen Glasfritten und Rauhigkeitsadditive in den angegebenen Anteilen gemischt und weisen die angegebenen mittleren Korngrößen auf. Die thermische Ausdehnung der eingesetzten Rauhigkeitsadditive ist ebenfalls in Tabelle 2 angegeben.

Tabelle 2:

| Zusammensetzungen und Eigenschaften von Druckfarben und damit Hergestellten Konusbeschichtungen; erfindungsgemäße Beispiele Nr. 1 - 5,Vergleichsbeispiel Nr. 6 | | | | | | |
|---|---|---|---|---|---|---|
| Beispiel | 1 | 2 | 3 | 4 | 5 | 6 |
| Glas-Nr. | 1 | 1 | 2 | 2 | 3 | 4 |
| Glasfritte | | | | | | |
| Anteil [Gew.%] | 41 | 41 | 55 | 55 | 40 | 42 |
| Korngröße d$_{50}$ [μm] | 2,5 | 2,5 | 2,1 | 2,1 | 3,5 | 2,5 |
| | | | | | | |
| Rauhigkeitsadditiv | SiO$_2$-Glas | SiO$_2$-Glas | Zirkonsilikat | Zirkonsilikat | Korund | BaSO$_4$ |
| Anteil [Gew.%] | 3 | 3 | 9 | 9 | 8 | 8 |
| Korngröße d$_{50}$ [μm] | 1 | 1 | 1,4 | 1,4 | 1,5 | 1 |
| Therm. Ausdehnung von 20 bis 300 °C [10$^{-6}$/K] | 0,6 | 0,6 | 4,5 | 4,5 | 9 | |
| Paste Ölzusatz [Gew.%] | 56 | 56 | 36 | 36 | 52 | 50 |
| | | | | | | |
| Einbrand | | | | | | |
| T$_{max}$[°C] | 590 | 610 | 570 | 610 | 590 | 570 |
| | | | | | | |
| Eigenschaften Konusbeschichtung | | | | | | |
| Mittlere Schichtdicke [μm] | 4,5 | 4 | 6 | 7 | 8 | 10 |

(fortgesetzt)

| Rauhigkeit $R_q$ rms [$\mu$m] | 0,68 | 0,69 | 1,25 | 1,15 | 1,22 | 1,02 |
|---|---|---|---|---|---|---|
| | | | | | | |
| Visuelle Begutachtung | | | | | | |
| Risse | keine | keine | keine | keine | keine | vereinzelt |
| Abplatzungen | keine | keine | keine | keine | keine | keine |
| | | | | | | |
| Schichthaftung | + | + | + | + | + | + |
| Dichtigkeit | + | + | + | + | + | + |
| Chemische Beständigkeit | + | + | + | + | 0 | + |
| +: ohne Einschränkung bestanden<br>0: mit Einschränkung bestanden<br>-: nicht bestanden | | | | | | |

Die Pulvermischungen wurden mit Zusatz von organischen Anpastmitteln auf Ölbasis zu einer Paste verarbeitet. Abgestimmt auf die Dichten der Glasfritte wurden verschiedene Ölmengen zugesetzt, um Viskositäten von ca. 0,4 Pa·s bei 35°C einzustellen. Pulvermischung und Öl addieren sich in Tabelle 2 zu 100 Gew.%. Zur Homogenisierung der Pasten wurden diese auf einem Dreiwalzenstuhl behandelt.

Mit den erhaltenen Pasten wurden Glasspritzen aus einem Borosilikatglas des Typs FIOLAX® und mit einer thermischen Ausdehnung von 4,9·10$^{-6}$/K im Konusbereich beschichtet. Die Bedruckung des Glaskonus mit den Pasten erfolgte mit einer Beschichtungswalze. Die Breite der umlaufenden Konusbeschichtung betrug 5 mm. Die beschichteten Glasspritzen wurden in einem Durchlaufofen mit den in Tabelle 2 angegebenen Einbrandtemperaturen für eine Zeitdauer von ca. 3 Minuten eingebrannt. In Tabelle 2 sind außerdem die nach dem Einbrand erhaltenen mittleren Schichtdicken und Rauhigkeitswerte angegeben. Die Rauhigkeitswerte und die Schichtdicke wurden mit einem Weißlicht-Interferometer der Firma Zygo bestimmt.

Die so erhaltenen Beschichtungen sind transparent bis transluzent und für die visuelle Kontrolle auf Unterschiede in der Rauhigkeit, Schichtdicke und Geometrie der Beschichtung gut sichtbar. Die Konusbeschichtungen wurden mit dem bloßen Auge und unter dem Lichtmikroskop auf Risse und Abplatzungen untersucht.

Die Haftfestigkeit der Beschichtung wurde im Kratztest mit einem Metalllineal bewertet. Bei der Prüfung wird ein Metalllineal mit der Kante über die bedruckte Fläche gekratzt. Die Prüfung gilt als bestanden, wenn dabei keine Schichtbestandteile abgeschabt wurden. Die in dieser Weise getesteten Bereiche wurden anschließend im Lichtmikroskop untersucht.

Die Dichtigkeit wurde an sterilisierten (für 20 Minuten bei 121 °C) Glasspritzen untersucht. Die Glasspritzen werden dabei am Konus mit handelsüblichen Dichtkappen aus Kunststoff verschlossen und mit destilliertem Wasser gefüllt. Im Innern wird ein Druck von 1 bar angelegt und für 30 Sekunden aufrecht gehalten. Um die Untersuchung zu bestehen, darf dabei keine Flüssigkeit austreten und die Verschlusskappen dürfen nicht abspringen.

Bei der Messung der chemischen Beständigkeit wurden die beschichteten Glasspritzen für 5 Minuten in ein Säurebad aus verdünnter Salzsäure mit einem pH-Wert von 2 gelegt. Danach wurden die Glasspritzen unter Wasser abgespült und die Konusbeschichtung wurde mit dem bloßen Auge und unter dem Lichtmikroskop auf sichtbare Veränderungen geprüft. Es zeigte sich, dass nur die beschichtete Glasspritze nach Beispiel 5 der Tabelle 2 eine visuell erkennbare Veränderung der Konusbeschichtung aufwies. Nach der Säurebehandlung wurde erneut ein Dichtigkeitstest durchgeführt. Hier gab es bei keinem der Ausführungsbeispiele Beanstandungen. Es zeigte sich damit, dass die Glasfritte von Beispiel 5 hinsichtlich ihrer chemischen Beständigkeit grenzwertig ist. Die Forderungen an die Dichtigkeit wurden bestanden, aber visuell ist ein chemischer Angriff zu erkennen. Bei Anwendungen bei denen es auf eine hohe chemische Beständigkeit ankommt, sollte dieser Beschichtungstyp deshalb nicht ausgewählt werden.

Für jeden Test wurden mindestens 10 mit dem gleichen Material beschichtete Glasspritzen geprüft.

**Patentansprüche**

1. Spritze (1) mit einem Zylinder (2) zur Aufnahme einer Flüssigkeit und einem Konus (3) aus Borosilikatglas mit einer thermischen Ausdehnung zwischen 20 und 300 °C von weniger als 6·10$^{-6}$/K, wobei der Konus (3) einen Auslasskanal (4) umschließt und die Außenseite des Konus (3) zumindest bereichsweise eine Beschichtung (5) aufweist, die eine Glasmatrix mit darin eingebetteten strukturbildenden Partikeln aufweist, **dadurch gekennzeichnet,**
**dass** die Beschichtung (5) eine gegenüber der unbeschichteten Konusoberfläche erhöhte Rauhigkeit von Rq(rms) = 0,3 - 2 $\mu$m aufweist, und frei von Pb, Cd, Hg und $Cr^{VI}$ ist, und
**dass** die Spritze (1) eine hohe Dichtigkeit gegen den Austritt von Fluiden aufweist, bestimmt durch Befüllung mit Wasser und einer Beaufschlagung von einem Druck von 1 bar, wobei es während 30 Sekunden zu keinem Austritt von Fluiden oder einem Abspringen der Verschlusskappen kommt.

2. Spritze nach Anspruch 1 mit einer Rauhigkeit der Beschichtung (5) von $R_q$(rms) = 0,5 - 1,8 $\mu$m.

3. Spritze nach Anspruch 1 oder 2, **gekennzeichnet durch** eine mittlere Schichtdicke der Beschichtung (5) von 0,5 bis 20 $\mu$m.

4. Spritze nach mindestens einem der vorhergehenden Ansprüche, **dadurch gekennzeichnet, dass** die Glasmatrix unterhalb von 700 °C eine Viskosität von $\leq 10^6$ dPas erreicht.

5. Spritze nach mindestens einem der vorhergehenden Ansprüche, **dadurch gekennzeichnet, dass** die strukturbildenden Partikel keramische oder Hartglaspartikel sind, die eine mittlere Korngröße $d_{50}$ von 0,2 bis 5 $\mu$m besitzen.

6. Spritze nach mindestens einem der vorhergehenden Ansprüche, **dadurch gekennzeichnet, dass** die strukturbildenden Partikel an der Beschichtung (5) einen Anteil von 1 - 30 Gew.% haben.

7. Spritze nach mindestens einem der vorhergehenden Ansprüche, **dadurch gekennzeichnet, dass** das Material der strukturbildenden Partikel im Temperaturbereich von 20 bis 300 °C eine thermische Ausdehnung von weniger als $8 \cdot 10^{-6}$/K besitzt.

8. Spritze nach mindestens einem der vorhergehenden Ansprüche, **gekennzeichnet durch** eine thermische Ausdehnung der Glasmatrix der Konusbeschichtung von kleiner als $7,5 \cdot 10^{-6}$/K, bevorzugt kleiner als $7 \cdot 10^{-6}$/K, im Temperaturbereich zwischen 20 und 300 °C.

9. Spritze nach mindestens einem der vorhergehenden Ansprüche, **dadurch gekennzeichnet, dass** die Glasmatrix der Konusbeschichtung (5) 40 - 75 Gew.% $Bi_2O_3$, 3 - 20 Gew.% $B_2O_3$ und 10 - 30 Gew.% $SiO_2$ aufweist.

10. Spritze nach einem der Ansprüche 1 bis 8, **gekennzeichnet dadurch, dass** die Glasmatrix der Konusbeschichtung (5) 15 - 48 Gew.% ZnO, 8 - 40 Gew.% $B_2O_3$ und 8 - 52 Gew.% $SiO_2$ aufweist.

11. Spritze nach mindestens einem der vorhergehenden Ansprüche, **dadurch gekennzeichnet, dass** die Beschichtung (5) auf der Außenseite des Konus symmetrisch umlaufend ausgeführt ist und 2 - 10 mm breit ist.

12. Spritze nach mindestens einem der vorhergehenden Ansprüche, **dadurch gekennzeichnet, dass** die thermische Ausdehnung des Borosilikatglases zwischen 20 °C und 300 °C $4,5 \cdot 10^{-6}$/K bis $5,5 \cdot 10^{-6}$/K beträgt, bevorzugt < $5,0 \cdot 10^{-6}$/K.

13. Spritze nach wenigstens einem der vorhergehenden Ansprüche, **dadurch gekennzeichnet, dass** die Spritze in Summe weniger als 100 ppm Pb, Cd, Hg und $Cr^{VI}$ enthält.

**Claims**

1. A syringe (1) comprising a cylinder (2) for holding a liquid and a cone (3) made of borosilicate glass with a thermal expansion between 20 and 300 °C of less than $6 \cdot 10^{-6}$/K, wherein the cone (3) encloses an outlet passage (4) and the outer surface of the cone (3) has a coating (5), at least in some areas thereof, which comprises a glass matrix with texture-defining particles embedded therein, **characterized in**

that the coating (5) has an increased roughness of Rq (rms) = 0.3 - 2 $\mu$m compared to the non-coated cone surface, and is free of Pb, Cd, Hg and $Cr^{VI}$; and
that the syringe (1) exhibits a high level of tightness against escape of fluids, as determined by filling it with water and applying a pressure of 1 bar without showing any escape of fluids or closure caps coming off for 30 seconds.

2. The syringe according to claim 1, with a roughness of the coating (5) of Rq (rms) = 0.5 - 1.8 $\mu$m.

3. The syringe according to claim 1 or 2, **characterized by** an average layer thickness of the coating (5) from 0.5 to 20 $\mu$m.

4. The syringe according to at least one of the preceding claims, **characterized in that** the glass matrix reaches a viscosity of $\leq 10^6$ dPa·s below 700 °C.

5. The syringe according to at least one of the preceding claims, **characterized in that** the texture-defining particles are ceramic or hard glass particles which have a mean grain size $d_{50}$ from 0.2 to 5 $\mu$m.

6. The syringe according to at least one of the preceding claims, **characterized in that** the texture-defining particles account for a proportion of 1 - 30 wt.% of the coating (5).

7. The syringe according to at least one of the preceding claims, **characterized in that** the material of the texture-defining particles exhibits thermal expansion of less than $8 \cdot 10^{-6}$/K in the temperature range from 20 to 300 °C.

8. The syringe according to at least one of the preceding claims, **characterized by** a thermal expansion of the glass matrix of the cone coating of less than $7.5 \cdot 10^{-6}$/K, preferably less than $7 \cdot 10^{-6}$/K, in the temperature range between 20 and 300 °C.

9. The syringe according to at least one of the preceding claims, **characterized in that** the glass matrix of the cone coating (5) comprises 40 - 75 wt.% of $Bi_2O_3$, 3 - 20 wt.% of $B_2O_3$ and 10 - 30 wt.% of $SiO_2$.

10. The syringe according to any one of claims 1 to 8, **characterized in that** the glass matrix of the cone coating (5) comprises 15 - 48 wt.% of ZnO, 8 - 40 wt.% of $B_2O_3$ and 8 - 52 wt.% of $SiO_2$.

11. The syringe according to at least one of the preceding claims, **characterized in that** the coating (5) on the outer surface of the cone is symmetrical circumferentially and has a width of 2 - 10 mm.

12. The syringe according to at least one of the preceding claims, **characterized in that**, between 20 °C and 300 °C, the borosilicate glass has a thermal expansion of $4.5 \cdot 10^{-6}$/K to $5.5 \cdot 10^{-6}$/K, preferably $< 5.0 \cdot 10^{-6}$/K.

13. The syringe according to at least one of the preceding claims, **characterized in that** the syringe contains less than 100 ppm of Pb, Cd, Hg and Cr$^{VI}$ in total.

**Revendications**

1. Seringue (1) comportant un cylindre (2) destiné à la réception d'un liquide et un cône (3) à base de verre de borosilicate ayant une dilatation thermique entre 20 et 300 °C de moins de $6.10^{-6}$/K, le cône (3) comprenant un canal d'évacuation (4) et la face externe du cône (3) comportant au moins par zones un revêtement (5) qui comporte une matrice de verre avec des particules structurantes incorporées dans celle-ci,
**caractérisée en ce que**
le revêtement (5) présente une rugosité, accrue par rapport à la surface non revêtue du cône, de $R_q$(rms) = 0,3 - 2 $\mu$m, et est exempt de Pd, Cd, Hg et Cr$^{VI}$, et
**en ce que** la seringue (1) présente une haute étanchéité vis-à-vis de la fuite de fluides, déterminée par remplissage avec de l'eau et une application d'une pression de 1 bar, aucune fuite de fluides ni éjection des capuchons de fermeture ne se produisant pendant 30 secondes.

2. Seringue selon la revendication 1, ayant une rugosité du revêtement (5) de $R_q$(rms) = 0,5 - 1,8 $\mu$m.

3. Seringue selon la revendication 1 ou 2, **caractérisée par** une épaisseur moyenne de couche du revêtement (5) de 0,5 à 20 $\mu$m.

**4.** Seringue selon au moins l'une quelconque des revendications précédentes, **caractérisée en ce que** la matrice de verre atteint au-dessous de 700 °C une viscosité de $\leq 10^6$ dPa.s.

**5.** Seringue selon au moins l'une quelconque des revendications précédentes, **caractérisée en ce que** les particules structurantes sont des particules de verre trempé ou céramiques, qui ont une taille moyenne de grain $d_{50}$ de 0,2 à 5 $\mu$m.

**6.** Seringue selon au moins l'une quelconque des revendications précédentes, **caractérisée en ce que** les particules structurantes constituent 1 à 30 % en poids du revêtement (5).

**7.** Seringue selon au moins l'une quelconque des revendications précédentes, **caractérisée en ce que** le matériau des particules structurantes présente dans la plage de température de 20 à 300 °C une dilatation thermique de moins de $8.10^{-6}$/K.

**8.** Seringue selon au moins l'une quelconque des revendications précédentes, **caractérisée par** une dilatation thermique de la matrice de verre du revêtement du cône de moins de $7,5.10^{-6}$/K, de préférence moins de $7.10^{-6}$/K, dans la plage de température comprise entre 20 et 300 °C.

**9.** Seringue selon au moins l'une quelconque des revendications précédentes, **caractérisée en ce que** la matrice de verre du revêtement (5) du cône comporte 40 - 75 % en poids de $Bi_2O_3$, 3 - 20 % en poids de $B_2O_3$ et 10 - 30 % en poids de $SiO_2$.

**10.** Seringue selon l'une quelconque des revendications 1 à 8, **caractérisée en ce que** la matrice de verre du revêtement (5) du cône comporte 15 - 48 % en poids de ZnO, 8 - 40 % en poids de $B_2O_3$ et 8 - 52 % en poids de $SiO_2$.

**11.** Seringue selon au moins l'une quelconque des revendications précédentes, **caractérisée en ce que** le revêtement (5) a une disposition périphérique symétrique sur la face externe du cône et une largeur de 2 - 10 mm.

**12.** Seringue selon au moins l'une quelconque des revendications précédentes, **caractérisée en ce que** la dilatation thermique du verre de borosilicate entre 20 °C et 300 °C vaut de $4,5.10^{-6}$/K à $5,5.10^{-6}$/K, de préférence est $< 5,0.10^{-6}$/K.

**13.** Seringue selon au moins l'une quelconque des revendications précédentes, **caractérisée en ce que** la seringue contient au total moins de 100 ppm de Pd, Cd, Hg et Cr$^{VI}$.

Figur 1:     Längsschnitt durch den vorderen Abschnitt einer Glasspritze mit
Konus und einer Konusbeschichtung der Breite b

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4589871 A
- WO 2010150042 A
- US 5851201 A